# EUROPEAN PATENT SPECIFICATION

(11) **EP 3 103 446 B1**
(45) Date of publication and mention of the grant of the patent: **14.02.2024**
(21) Application number: 14872234.1
(22) Date of filing: 16.12.2014
(51) Int. Cl.: A61K 9/06, A61K 9/08, A61K 9/00, A61K 9/70, A61K 31/13, A61K 31/132, A61K 31/198, A61K 31/4415, A61K 31/28, A61K 33/24, A61K 38/02, A61K 38/05, A61K 38/06, A61P 27/10

(54) **PHARMACEUTICAL PREPARATION FOR PREVENTING AND TREATING PROGRESSIVE MYOPIA**
PHARMAZEUTISCHES PRÄPARAT ZUR VERHÜTUNG UND BEHANDLUNG VON PROGRESSIVER MYOPIE
COMPOSITION PHARMACEUTIQUE DESTINÉE À LA PRÉVENTION ET AU TRAITEMENT DE LA MYOPIE PERNICIEUSE

(30) Priority: 17.12.2013 RU 2013155972
(43) Date of publication of application: 14.12.2016
(73) Proprietor: Zakharov, Ivan Dmitrievich, Moscow 109125 (RU); Korigodskiy, Aleksandr Robertovich, Moscow 107113 (RU)
(72) Inventor: Zakharov, Ivan Dmitrievich, Moscow 109125 (RU); Korigodskiy, Aleksandr Robertovich, Moscow 107113 (RU)
(74) Representative: Osmans, Voldemars
(86) International application number: PCT/RU2014/000949
(87) International publication number: WO 2015/094019

(56) References cited:
- WO-A1-2013/027222
- WO-A1-2013/082565
- WO-A2-2007/011875
- CN-A- 1 781 493
- RU-C1- 2 012 336
- RU-C2- 2 192 261
- RU-C2- 2 192 261
- US-A- 4 248 855
- US-A- 4 797 423
- US-A- 5 075 105
- US-A- 5 075 105
- US-A1- 2003 036 535
- US-A1- 2003 087 830
- US-A1- 2004 137 069
- US-A1- 2004 248 962
- US-A1- 2005 085 415
- US-A1- 2006 110 459
- US-A1- 2011 130 388
- US-A1- 2013 233 201
- DATABASE WPI Week 200239 September 2002 (2002-09) Thomson Scientific, London, GB; AN 2002-362397 XP002772402, & WO 02/24191 A1 (YONGGUANG PHARMACY CO LTD) 28 March 2002 (2002-03-28)
- Anonymous: "Phenylephrine Hydrochloride Ophthalmic Solution, USP 2.5% and 10%. (PRESCRIBING INFORMATION)", , March 2013 (2013-03), pages 1-5, XP055392795, Retrieved from the Internet: URL:https://www.accessdata.fda.gov/drugsat fda_docs/label/2013/203510s000lbl.pdf [retrieved on 2017-07-20]

## Description

### Technical field

The invention relates to medicine, in particular to ophthalmology, and is intended to strengthen the sclera in case of progressive myopia.

### BACKGROUND OF THE INVENTION

Progressive myopia is one of the most urgent problems of modern ophthalmology. Despite some progress made in the prevention and treatment of this disease, it often leads to irreversible changes in the eye fundus and a significant decrement in visual acuity in the young working age, which causes significant restrictions in professional work or even physical disability (see Libman E.S., Ryazanov D.P., Kaleyeva E.V. "Disability as a result of visual impairment in Russia". V Russian national ophthalmology forum. Collection of scientific works of the scientific-practical conference with participants from abroad. Moscow, 2012, 2, 797-798 - [1]).

In this regard, the development of new effective methods of pathogenetically oriented therapy of this eye disease is a very important scientific and practical problem.

It is now established that one of the leading pathogenetic factors of the onset and progression of myopia (nearsightedness) is stretching and weakening of the scleral shell of the eye associated with the development of degenerative process in its connective tissue, accompanied by elongation of the eyeball in the anterior-posterior direction (see Avetisov E.S. "Myopia". Moscow, 1999, 285 p. - [2]; Iomdina E.N. "The biomechanical and biochemical sclera disorders with progressive myopia, and methods of their correction". Visual function in children and their correction"/ Edited by Avetisov S.E., Kashchenko T.P., Shamshinova A.M. Moscow, 2006, 163-183 - [3]; Curtin B.J. «The Myopias: Basic science and clinical management». Harper and Row. Philadelphia. 1985 - [4]). This process is manifested in the fibrillar structures degradation, insoluble collagen content reduction and increased soluble fractions level in the sclera with the growth of myopic refraction, that shows a certain immaturity of sclera collagen structures and the disassimilation predominance over the synthesis process. This imbalance is largely due to inadequate formation of intra- and intermolecular bonds (cross-links), stabilizing scleral collagen (see Bailey A.J. «Intermediate labile inter molecular cross-links in collagen fibrils». Biochim. Biophys. Acta. 1968. v.160, 447-453 - [5]). It is known that cross-links are crucially important in forming the optimal biomechanical tissue characteristics, so the identified reduction of sclera collagen transverse coherence with progressive myopia is a major factor leading to a weakening of its supporting function and as a consequence to the anterior-posterior axis of the eye lengthening and the progression of myopia [3].

In this regard, various methods of strengthening this layer of the eye have been developed. In particular, the widely used surgical techniques are also strengthening the sclera with the use of different plastic materials (donor and synthetic), which were located around the eyeball, capturing the eyeball posterior pole, providing its mechanical support and partially stimulating scleral tissue with grafts disassimilation products. (cm. E.P. Tarutta. "Sclera strengthening treatment and prevention of progressive myopia complications." Visual function in children and their correction"/ Edited by Avetisov S.E., Kashchenko T.P., Shamshinova A.M. Moscow, 2006, 191-202 - [6]; Ward B., Tarutta E. «The efficacy and safety of posterior pole buckles in the control of progressive high myopia». Eye (Lond). 2009 Dec; 23 (12): 2169-74 - [7]).

The disadvantage of existing sclera strengthening interventions is that a clinically significant effect (length of the anterior-posterior axis of the eye and refraction stabilization) is limited mostly by 1-2 years, and then (in the long-term follow) myopia progression resumes [6]. This is due to the fact that the structure of myopic sclera remains pathologically changed after treatment, and the supporting implant properties are weaken with time.

To strengthen the weak myopic sclera, a method comprising administering under the Tenon's capsule (fascia) on the posteroexternal surface of the sclera the expandable polymer gel composition based on the dry ingredient, liquid water-containing component, 3% solution of hydrogen peroxide and a complex compound of copper and pyridoxine - dichloride [2-methyl-3-oxy-4,5-di(oxymethyl)-pyridine] copper (II) (INN - "Cupir"), the content of which in the composition is 0,11-0,31 wt.%.(see Patent of the Russian Federation 2012336, 1987/1994. "The composition for the treatment of progressive myopia". Avetisov E.S., Vinetskaya M.I., Iomdina E.N. et al. - [8]). The resulting foamed composition was replaced during the gradual degradation by newly formed connective tissue (capsule) on the sclera surface, resulting in a new formed biocomposite "sclera-connective tissue". After 6 months after the Cupir composition administration, the formed capsule thickness is 146 microns on average, while after the administration of the polymeric composition without Cupir - 109 microns. At the same biomechanical properties of the newly formed complex "sclera-connective tissue" only marginally affected and not statistically significant: since the modulus of elasticity (the most important parameter showing the sclera strengthening effect) after the administration of the composition with Cupir is 27.1 ± 2.3 MPa, and after the administration of the composition without Cupir is 25.3 ± 1.9 MPa, i.e. it increases only by 7.1%.

Furthermore, the foaming process of the composition can lead to irreproducible results, and the foam composition itself, which is located under the Tenon's capsule of at least 12 months until complete resorption, can significantly injure adjacent eye tissues.

This technical decision is taken as the closest analogue.

In prior art, there is no information on the use of sclera strengthening drugs administered non-invasively, i.e. directly in the eye conjunctival sac (for example, by instillation).

The outer shell of the connection - the conjunctiva, as we know, has a well-developed circulatory and lymphatic systems that reliably protect the sclera located under the conjunctiva from penetration of most chemical compounds (see L.V. Shilnikov. Encyclopedia of Clinical Ophthalmology. 2013 Scientific book, 380 p. - [9]).

For this reason, the external layer of the eye - conjunctiva - and Tenon's shell is virtually impermeable for most drugs, especially for water-soluble polar substances. Only a small amount of low-polar lipophilic substances that poorly soluble in water, for example, corticosteroids, are able to penetrate through this barrier (see Morozov V.I., Yakovlev A.A. Pharmacotherapy of eye diseases: a handbook. Publ. 3rd. M .: Medicine, 1998 - 336 p. - [10]).

Therefore, the functional properties of medicinal products intended for sclera strengthening and administration by instillation, besides their effective sclera strengthening action must also have the ability to effectively penetrate through the outer shell of the conjunctiva and land on the scleral tissue.

### Summary of the invention

The task of the proposed invention is to provide a ready-to-use pharmaceutical product (hereinafter - the drug, the pharmaceutical), contributing substantial and rapid increase in the elastic-strength characteristics of the sclera with non-invasive administration into conjunctival cavity (which can be used for prevention and treatment of progressive myopia), for example, in the form of a solution, gel or ophthalmic medicated film (hereinafter - OMF), i.e. drug that can penetrate into the sclera directly through the conjunctiva and Tenon's membrane.

Another task is to provide a drug which main components would be preferably products of natural origin or derived from such products and, accordingly, would be characterized by a minimal immune response and side effects less likely than known from the prior art analogues.

The technical results of the use of the claimed solution are:
- strengthening the sclera with progressive myopia and reduction of duration of such strengthening by activating the cross-linking of the sclera collagen molecules, with the exception of traumatic, toxic and inflammatory effects on the eyeball and the surrounding tissue of the orbit in the treatment of progressive myopia.
- easy and non-invasive treatment - the usual way of instillation, using eye gel or ophthalmic medicated film;
- no toxic or inflammatory effects throughout the course of drug treatment;
- no foreign body is entered in the area of the orbit and situated therein for a long time (foam), which excludes its possible damaging and irritating effect on the eyeball and the surrounding tissues of the orbit.

The technical results are achieved through a pharmaceutical preparation for use in the prevention and treatment of progressive myopia, wherein the preparation is in the form of an aqueous solution for instillation or an ophthalmic gel or ophthalmic medicated film dosage containing in its composition an effective amount of primary amine containing at least two primary amine groups in the form of a salt, or in a complex compound of a transition metal, or mixtures thereof.

Used amines with functional groups (hereinafter referred to as AFG) according to the claimed invention have in their composition at least two primary amine groups in the form of salt or in composition of a complex compound of transition metal or mixtures thereof which are reactive and may participate in the collagen molecules cross-linking processes. There must be at least two of such groups in the amine molecule that acts as a cross-linking agent.

A particular criterion in choosing these materials is their minimal toxicity or non-toxicity, therefore most of the compositions used are of natural origin. In accordance with the claimed invention amino acids, oligopeptides, polypeptides, polyamines, or a mixture thereof can be used as amine functional groups, provided they are containing at least two primary amine groups.

The known natural and synthetic amino acids containing two or more amino groups, such as: 2,4-diaminobutanoic acid or 2,5-diaminopentanoic acid (ornithine), or 2,6-diaminohexanoic acid (lysine), or 2,6-diamino-5-hydroxyhexanoic acid (hydroxylysine), or 2,7- diaminoheptanoic acid, or 2, 8 diaminooctanoic acid, or 2,9-diaminononanoic acid, or 2,10-diaminodecanoic acid, or 2,12-diaminododecanoic acid, or a mixture thereof are used as amino acids.

There may be used D-, L- (natural) optical isomers or D, L-mixture (racemate) of these amino acids.

The heteromerous oligopeptides, having at least one link of L-ornithine, L-lysine or L-hydroxylysine, for example, dipeptides: L-alanine-L-lysine, L-arginine-L-lysine, L-valine-L-lysine, L-hydroxylysine-L-lysine, L-hydroxyproline-L-lysine, L-histidine-L-lysine, glycine-L-lysine, L-isoleucine-L-lysine, L-leucine-L-lysine, L-methionine-L-lysine, L-proline-L-lysine, L-serine-L-lysine, L-tyrosine-L-lysine, L-threonine-L-lysine, L-tryptophan-L-lysine, L-phenylalanine-L-lysine, L-cysteine-L-lysine, or tripeptides: L-alanine-L-histidine-L-lysine, L-arginine-L-histidine-L-lysine, L-valine-L-histidine-L-lysine, L-hydroxylysine-L-histidine-L-lysine, L-hydroxyproline-L-histidine-L-lysine, L-histidine-L-histidine-L-lysine, glycine-L-histidine-L-lysine, L-isoleucine-L-histidine-L-lysine, L-leucine-L-histidine-L-lysine, L-methionine-L-histidine-L-lysine, L-proline-L-histidine-L-lysine, L-serine-L-histidine-L-lysine, L-tyrosine-L-histidine-L-lysine, L-threonine-L-histidine-L-lysine, L-tryptophan-L-histidine-L-lysine, L-phenylalanine-L-histidine-L-lysine, L-cysteine-L-histidine-L-lysine, or a mixture thereof are used as oligopeptides.

The homomerous compounds derived from amino acids containing two amino groups: poly-L-omithine, poly-L-lysine, poly-L-hydroxylysine, poly-L-arginine or a mixture thereof are used as polypeptides. The compounds having a molecular weight from 300 (corresponding to a dimer) to 30000 are used.

The natural or modified natural compounds containing 2-4 basic amino groups or may be used as the polyamines, in particular:
- diamines: putrescine (1,4-diaminbutane), 1,4-piperazine, 2-putrescine, cadaverine (1,5-pentamethylenediamine), 1,8-diaminooctane, 1,12-diaminododecane, 2,2'-(ethylenedioxy)bis(ethylamine), 4,9-dioxa-1,12-dodecanediamine, 3,6,9-trioxa-1,11-undecandiamine, 4,7, 10- trioxa -1, 13-tridecandiamine;
- spermidine and its derivatives: 1-methylspermine, 2-methylspermidine, 3-methylspermidine, 8-methylspermidine, 1,1-dimethylspermidine, 2,2-dimethylspermidine, 3,3-dimethylspermidine, 5,5-dimethylspermidine, 5,8-dimethylspermidine, 8,8-dimethylspermidine, 2-hydroxyspermidine, homospermidine;
- spermine and its derivatives: spermine, 1-methylspermine, 6-methylspermine, 1,12-dimethylspermine, 5,8-dimethylspermine, 1,1,12,12-tetramethylspermine, 3,3,10,10 - tetramethylspermine, norspermine, or a mixture thereof.

The above mentioned oligopeptides and polypeptides, and the polyamines can be used in lower concentrations than the concentrations intervals claimed below because of its high efficiency. This concentration may be 0.05-0.3 wt.% for different types of pharmaceutical products. However, they are preferably used in combination with other AFG, such as amino acids, so that the total concentration of both types of cross-linkers was in the range claimed.

AFG containing basic amino groups and aqueous solutions in its composition are alkaline. Therefore, their use is possible only in the neutralized form: in the form of salt or complex compound with the transition metal cation. Such salt can be obtained by neutralizing the amine with functional groups with the acid.

In accordance with the present invention, the low molecular weight acids of synthetic or natural origin, having one or more acid groups and forming non-toxic or low-toxic salts, for example: inorganic - hydrochloric, hydrobromic, nitric, sulfuric, phosphoric acid or organic acids - acetic, or dichloracetic, or pivalic (trimethylacetic), or glycolic, or lactic, or glutamic, or pyroglutamic, or asparagic acid, or ascorbic, or valerianic, or pelargonic, or capric or undecylic, or lauric, or myristic, or undecylenic, or sorbic, or pyruvic acid, or succinic, or fumaric, or maleic, or adipic, or pimelic, or sebacic, or azelaic acid, or malic, or tartaric, or citric acid, mucic (galactaric), or nicotinic (vitamine PP), or salicylic, or phenylacetic, or 2-[(2,6-dichlorophenyl)amino]benzeneacetic (Diclofenac) or Gallic (3,4,5-trihydroxybenzoic acid), or pantoic, or pantothenic (vitamine B₃) or folic (vitamine B₉), or asiatic, or madecassic acid, or a mixture thereof are used as acids to neutralize AFG.

In addition to low molecular weight acids, the polymeric acid can be used to neutralize the AFG: polyacrylic acid or copolymers of acrylic acid or a mixture thereof, also the methacrylic, maleic, itaconic acid, N-vinylpyrrolidone or a mixture thereof can be used as the copolymers of acrylic acid.

These polymeric acids have a molecular weight of 1000-30000. Due to such a relatively low molecular weight, these polymers can penetrate through the conjunctiva and Tenon's membrane, as well as can be easily removed from the body.

Dibasic or more low-molecular acids and polymer acids may also be used in partially neutralized form, which is obtained when neutralized with a base to a degree of neutralization of not more than 0.5. With this, alkali and alkaline earth metals (Na, K, Ca, Mg) hydroxides and amines: ammonia, primary, secondary, tertiary organic amines, or mixtures thereof are used as a base.

It is known that in eyes with high myopia (in equatorial and posterior sections of the sclera, and in the choroid) there was revealed a significant reduction in levels of zinc, copper, iron and other micronutrients involved in metabolism of connective tissue, particularly during the formation of the stabilizing cross-linking in the collagen fiber and in the functioning of the antioxidant defence system (see E.N. Iomdina. "Biomechanics of the scleral shell of the eye with myopia: diagnostics of defects and their experimental correction". Thesis of doctor of biological sciences. Moscow, 2000, 316 p. - [11]). An imbalance of these micronutrients, associated with the violation of the sclera structure under myopia, is one of the major factors contributing to the change of biomechanical characteristics of the sclera, i.e. weakening its support properties. Therefore, the introduction of transition metal compounds (copper and zinc) along with AFG into the composition is physiologically appropriate to strengthen the sclera tissue.

The best form of using these transition metals according to the claimed invention is their use in immobilized form - as complex compounds with amines used. These transition metal cations react with basic amines, as well as amino acids, giving a stable neutral complexes (coordination compounds) (see "Coordination chemistry" Skopenko V.V., Tsivadze A.Y., Savranskii L.I., Garnovskii A.D. Moscow: ECC "Akademkniga", 2007, 487 p. - [12]).

It is known that in complex compounds the central atom (complexing agent) connects one or more ligand molecules, depending on the coordination number of complexing agent and the ligand denticity (the number of functional groups in the ligand - the binding sites).

Amines with functional groups reacting with the transition metal cations normally act as bidental ligands. At the same time, the cations Cu²⁺ and Zn²⁺ have a coordination number of 4, therefore during the interaction with amine functional groups, they form a stable complex compound with two molecules of ligand. (see Conato C., Contino A., Maccarrone G., Magr A., Remelli M., Tabb G. Copper (II) complexes with l-lysine and l-omithine: is the side-chain involved in the coordination. A thermodynamic and spectroscopic study. Thermochimica Acta, 362, number 1, 2000, 13-23. - [13]; N.G. Furmanova, J.I. Berdalieva, T.S. Chernaya, V.F. Resnyanskii, N.K. Shyitieva, K.S. Sulaimankulov. Synthesis and crystal structures of pyridoxine coordination compounds with zinc and cadmium sulphates. Crystallography. 2009, 54, No. 2, 255-261 - [14]).

These transition metals - cations Cu²⁺ and Zn²⁺ are used in form of salts with the following anions: chloride, bromide, nitrate, sulfate, acetate, glycolate, lactate, or mixtures thereof.

All listed AFG compounds (compounds that cross-link collagen) are forms of active substance used in the composition of the claimed invention.

The present technical solution substantially represents the alternative forms of the drug:
- an aqueous solution intended for instillation (may contain a gellant for prolonging action);
- eye gel (containing cross-linked gelling agent) laid down for the lower eyelid;
- medicated film laid down for a lower eyelid.

### The claimed drug synthesis

Active ingredient is "salt or a complex compound of a transition metal based on amine with functional groups" wherein the amine is a primary amine containing at least two primary amine groups (AFG compound) can be synthesised in an aqueous medium and applied as a solution and may be recovered in dry form by removing the solvents used, and subsequently used for prepare respective solutions.

AFG compound can be obtained in ethanol or water-ethanol, preferably in concentrated form, and can be used without separation, for example, for the preparation of medicated films.

It is also noted that certain AFG compounds are manufactured as a finished reagent.

Furthermore, AFG compounds being melted can be processed together with a polymeric binder in ophthalmic medicated films, for example, by extrusion.

Substances consisting of AFG salts are prepared by the neutralization of the original AFG containing basic amino groups with the corresponding acid in the stoichiometric amount or close to it, as determined by necessary final pH. As the acids used for AFG neutralization in accordance with the claimed invention are weak for the most part, they should be used in amounts that excess the stoichiometric to achieve neutral pH. The reaction to obtain AFG salt is carried out in distilled water, isotonic solution, ethanol or a mixture thereof.

Such composition may also be obtained by dissolving in an aqueous medium the existing (preformed) salt of amine with functional groups and the acid, usually strong one, for example, hydrochloric. In this case, pH adjustment to the desired value can be done by adding the appropriate amounts of base, preferably an amine such as tris(hydroxymethyl)aminomethane, L-lysine or pyridoxine.

The low molecular weight compounds and polymeric acids are used to neutralize the AFG: polyacrylic acid or copolymers based on it, as well as their partially neutralized form or a mixture thereof.

Partially neutralized form of the polymer is obtained by polymer acid neutralization with the base to the extent of neutralization of not more than 0.5. With this, alkali metals (Na, K, Ca, Mg) hydroxides and amines: ammonia, primary, secondary, tertiary amines, or mixtures thereof are used as a base.

It is known, that the comfort of eye drops is greatly influenced by the pH value. Most of the eye drops has a pH in the range 5.0-9.0. At pH values > 9 and < 5.0, the eye drops cause a negative reaction (severe tearing and burning sensation).

The claimed pharmaceutical product in a solution, designed to strengthen the sclera, should have a pH of 5.5-8.0, preferably 7.0-7.4.

Drugs based on complex compounds of the transition metal is made during the interaction of AFG with the appropriate salt of the transition metal (Cu²⁺ or Zn²⁺), taken in stoichiometric amount or close to it, as determined by the desired end pH value. The reaction is conducted in an aqueous medium or ethanol. Such composition may also be obtained by dissolving the existing (preformed) complex compound based on AFG and appropriate transition metal salt in an aqueous medium.

The drug in form of the solution can be obtained on the basis of distilled water, isotonic solution, or mixtures thereof.

The saline (0.9 wt.% NaCl), a balanced saline BSS (Hank's solution) or aqueous solutions of non-ionic compounds such as glycerin, sorbitol, mannitol, propylene glycol, or dextrose, the concentration of which is preferably not more than 4 wt are used as an isotonic solution.%. In case of preparation of the compound in distilled water solution with a concentration of AFG less than isotonic, it is desirable to introduce the appropriate amount of isotonic additives (dry).

In case of the preparation of a pharmaceutical preparation in the form of ophthalmic solution and gel, the polymer gellant is used as an aqueous medium (previously prepared polymer solution with an appropriate concentration) or entered into an already prepared aqueous solution of AFG in concentrated form in an appropriate amount.

In case of the preparation of a pharmaceutical product as a solution, the soluble polymers are used. In case of the preparation of a pharmaceutical product in the form of eye gel, only cross-linked gels are used.

A method of producing a gellant based on cross-linked polyacrylic acid and its copolymers with C10-30-alkylacrylates with an acid reaction, the pre-dispersing of these polymer acids in water is needed with stirring and subsequent neutralization of the base, which are alkali metal hydroxides (Na, K) or amines: ammonia, primary, secondary and tertiary amines, including AFG, or mixtures thereof.

### Solution for instillation and eye gel

Pharmaceutical product in form of the water solution on the basis of AFG can be obtained on the basis of distilled water, isotonic solution, or mixtures thereof.

The saline (0.9 wt.% NaCl), a balanced saline BSS (Hank's solution) or aqueous solutions of non-ionic compounds such as glycerin, sorbitol, mannitol, propylene glycol, or dextrose are used as an isotonic solution. In case of preparation of the compound in distilled water solution with a concentration of AFG less than isotonic, it is desirable to introduce the appropriate amount of isotonic additives (dry).

In accordance with the present technical solution, the AFG active compound concentration in the solution for strengthening the sclera is preferably 0.15-7.0 wt.%. At higher concentrations, undesirable side effects are possible, such as irritation to the surrounding tissue. At lower concentrations the effect of the product use is insignificant.

In order to prolong its action, the claimed product in solution may contain polymeric gelling: hyaluronic acid, chondroitin sulphate, polyvinyl alcohol, methylcellulose, hydroxyethylcellulose, hydroxypropylcellulose, hydroxypropylguar, or a block copolymer polyethyleneglycol-polypropyleneglycol-polyethyleneglycol (e.g., Kolliphor^{®} P188 and P407, BASF), or poly-N-vinylpyrrolidone, or Na-carboxymethylcellulose. Those cross-linked gellants in the form of salts of cross-linked polyacrylic acid and its copolymers with C10-30-alcylacrylates (e.g., Carbopol^{®}. Noveon, Inc.) are used to produce ophthalmic gel with prolonged action.

The content of gellant in the pharmaceutical product is 0.1-5.0 wt.%. Such a wide range of concentrations and varying nature of the gellant allow to produce pharmaceuticals for the prevention and treatment of progressive myopia with different properties. Thus, a pharmaceutical product as a solution for instillation is prepared using the gellant in the uncross-linked form, or in its absence (see Table 1), and ocular gel (having greater higher viscosity than instillation solution) is prepared using a cross-linked gellant (see Table 2). It should be noted, that when using a gellant, the aqueous solution viscosity is determined not only by its content, but also by its molecular weight and its nature and largely if the cross-linked or uncross-linked form is used.

Preservatives can be added to the resulting solution after the introduction of the main components, such as chlorobutanol hydrate, cetylpyridinium chloride, benzalkonium chloride, paraoxybenzoic acid methyl ester (Nipagin), paraoxybenzoic acid propyl ester (Nipasol), as well as other additional ingredients.

**Table 1. Composition of a pharmaceutical product in the form of an aqueous solution to strengthen the sclera**

| Ingredient | Content, wt.% |
|---|---|
| AFG compound | 0.15-7.0 |
| gellant | 0-2.0 |
| isotonic agent | 0-4.0 |
| preservative and auxiliary components | 0-0.2 |
| distilled water | the rest |

**Table 2. Composition of a pharmaceutical product in the form of an eye gel to strengthen the sclera**

| Ingredient | Content, wt.% |
|---|---|
| AFG compound | 0.15-7.0 |
| gellant | 0.1-5.0 |
| isotonic agent | 0-4.0 |
| preservative and auxiliary components | 0-0.2 |
| distilled water | the rest |

### Ophthalmic medicated film

The use of pharmaceuticals in the form of ophthalmic medicated films are known to increase the efficiency of application and at the same time reduces the irritant or toxic effect on the eye, since the active ingredient of the drug, gradually being released from the film, is continuously and uniformly supplied in the conjunctiva and cornea, and the amount of it that drains into the nose with the tear fluid is low.

Important OMF properties are also stability, allowing to store it up to two years, high sterility and a small risk of infection, ease of laying in the conjunctival sac of both by medical personnel and by patients themselves.

The polymeric carriers for ophthalmic medicated films with various types of therapeutic activity must meet the following specific requirements:
- polymers should rapidly swell in tear fluid immediately after its placement in the conjunctival cavity to reduce the traumatic effect on the surrounding tissue;
- polymers should completely dissolve in the tear fluid to form viscous solutions that can encapsulate ocular tissue and hold for a long time on the surface thereof;
- polymers should be able to bind the active drug substance for a long time to provide sustained therapeutic effects.

In the present invention, the AFG compound is included in ophthalmic medicated films. Its content in the medicated films is preferably 5-50 wt.% (Table 3).

The different natural and synthetic polymers are used as a polymeric binder for the drug in this technical solution: polyacrylamide and copolymers thereof, polyvinyl-N-pyrrolidone and its copolymers, polyvinyl alcohol, Na-carboxymethylcellulose, hydroxypropylcellulose, sodium alginate, chitosan.

**Table 3. Composition of a pharmaceutical product in the form of an ophthalmic medicated film to strengthen the sclera**

| Ingredient | Content, wt.% |
|---|---|
| AFG compound | 3.0-30.0 |
| plasticizer and auxiliary components | 1.0-5.0 |
| polymeric carrier | the rest |

For the preparation of OMF, the joint solution is prepared based on the above-described AFG compound and a polymeric carrier in an aqueous, aqueous-alcoholic or alcoholic medium. Additionally, this solution can also be made with plasticizer and auxiliary components (e.g., surfactants). Thereafter, the resulting homogeneous solution of the components is poured onto the substrate and dried. The film of the desired shape and size is cut or punched from the formed polymer tape.

The drug is in the form of OMF can also be prepared in co-extrusion processing of AFG compounds and polymeric binder.

When used, the pharmaceutical product in form of OMF containing AFG compound is placed in the conjunctival sac and, wetting by the lachrymal fluid and swelling, it goes into elastic soft state and is held in the lower conjunctival arch until complete dissolution.

### Examples of claimed pharmaceutical products application

For strengthening the sclera using instillation, the drug solution was injected daily into the conjunctival sac of the left eye of "Chinchilla" breed rabbits, the right eye was kept as intact control. The treatment consisted of daily instillations, 2 times a day 1-2 drops within 1 month. During this period and for 1 month after the completion of instillation, the state of the rabbits eyes was controlled by biomicroscopy. No toxic or inflammatory effects were found. At the end of the observation period, the eyes were enucleated and used to prepare the samples of the sclera.

To strengthen the sclera of the eye with gel, the gel substance weighing 20-25 mg were laid with a spatula at a lower eyelid of the left eye of "Chinchilla" breed rabbits daily for 1 month, the right eye was kept as intact control. During this period and for 1 month after the completion of treatment, the state of the rabbits eyes was controlled by biomicroscopy. No toxic or inflammatory effects were found. At the end of the observation period, the eyes were enucleated and used to prepare the samples of the sclera.

For strengthening the sclera using ophthalmic medicated films, different types of film weighing 15 mg were laid in the lower conjunctival fornix of the left eye of "Chinchilla" breed rabbits daily for 1 month, the right eye was kept as intact control. During this period and for 1 month after the completion of instillation, the state of the rabbits eyes was controlled by biomicroscopy. No toxic or inflammatory effects were found. At the end of the observation period, the eyes were enucleated and used to prepare the samples of the sclera.

### Biomechanical performance of mammal sclera after application of the claimed drug

To determine the sclera biomechanical parameters - (tensile load P (N), tensile strength σ (MPa), elongation at break ε (%) and elasticity modulus E (MPa), the samples of standard width 4.0 mm were excised from these tissues with special knife with two cutting surfaces. After measuring their thickness on the PosiTector 6000 unit of company DeFelsko (USA), the samples were placed into the jaws of the deformation machine Autograph AGS-H of company Shimadzu (Japan), and mechanical tests were performed. Dependence "stress-strain", obtained in the sample stretching process (speed of 1 mm/min) until abruption, was continuously recorded by the computer unit in digital and graphic mode.

These sclera biomechanical testing data is given in Table 4.

**Table 4. Mechanical testing of the rabbits sclera after administration of pharmaceuticals**

| Example number | The concentration of AFG, wt.% | Mechanical properties of the sclera after treatment | | | | The ratio of mechanical properties after and before treatment | | | |
|---|---|---|---|---|---|---|---|---|---|
| | | P, N | σ, MPa | ε, % | E, MPa | P/Pₒ | σ/σ_{ο} | ε/εₒ | E/Eₒ |
| 1¹ | 1.0 | 16.3 | 12.7 | 23.0 | 34.2 | 1.10 | 1.22 | 0.90 | 1.14 |
| 2¹ | 3.3 | 23.8 | 14.7 | 29.8 | 38.1 | 1.24 | 1.40 | 0.69 | 1.38 |
| 3¹ | 2.0 | 17.4 | 19.9 | 25.7 | 28.5 | 1.62 | 1.29 | 0.95 | 1.42 |
| 4¹ | 0.5 | 24.0 | 8.8 | 25.0 | 29.7 | 1.29 | 1.30 | 0.70 | 1.19 |
| 5¹ | 0.15 | 19.5 | 11.9 | 31.4 | 36.2 | 1.14 | 1.20 | 0.68 | 1.12 |
| 6¹ | 7.0 | 22.5 | 22.7 | 35.4 | 31.9 | 1.69 | 1.46 | 0.70 | 1.72 |
| 7¹ | 2.0 | 18.9 | 24.0 | 28.7 | 29.6 | 1.78 | 1.49 | 0.83 | 1.34 |
| 8¹ | 4.0 | 17.0 | 15.7 | 34.7 | 40.3 | 1.46 | 1.51 | 0.75 | 1.51 |
| 9¹ | 4.0 | 24.0 | 13.9 | 23.8 | 42.1 | 1.21 | 1.40 | 0.69 | 1.56 |
| 10¹ | 2.0 | 21.2 | 10.5 | 36.2 | 34.6 | 1.60 | 1.27 | 0.91 | 1.30 |
| 11¹ | 2.0 | 18.3 | 20.1 | 26.6 | 39.3 | 1.33 | 1.41 | 0.87 | 1.26 |
| 12² | 1.0 | 21.7 | 23.4 | 28.9 | 45.1 | 1.66 | 1.25 | 0.71 | 1.18 |
| 13² | 1.5 | 16.9 | 13.4 | 38.4 | 35.8 | 1.85 | 1.39 | 0.63 | 1.39 |
| 14³ | 10.0 | 24.4 | 26.3 | 32.3 | 33.5 | 1.55 | 1.58 | 0.69 | 1.59 |

| | | | | | | | | | |
|---|---|---|---|---|---|---|---|---|---|
| ¹- water solution ²- ophthalmic gel ³- OMF | | | | | | | | | |

As the table shows, the sclera biomechanical properties significantly higher in case of the drug administration than the corresponding figures of the intact sclera. Increasing sclera tensile strength σ/σ_{ο} is at 1,20-1,58 times, while increasing the most important biomechanical criteria - modulus E/Eₒ of the sclera is at 1,12-1,72 times. This demonstrates the high efficiency of the drug used to strengthen the connective tissue of the sclera, including its ability to effectively penetrate through the outer shell of the conjunctival and land on the scleral tissue.

At the same time, the treatment of progressive myopia may be limited to a less pronounced strengthening effect. The degree of improving the strength at break and elasticity modulus can be reduced by decreasing the concentration of the components administered within the claimed range, or by reducing the number or instillation during the treatment.

### Estimated collagen cross-linking principle in case of the claimed drug administration

It is well known, that collagen fibrils (fibers) are stabilized by the system of cross-links (see [5] and Bailey A.J., Paul R.G., Knott L. «Mechanism of maturation and ageing of collagen». Mech. Ag. Dev. 1998, 106, 1-56 - [15]) and their formation is particularly important stage of formation of allysine (aldehyde derivative of lysine) by reaction of deamination of lysine or hydroxylysine side chains by the lysyl oxidase enzyme (copper-containing enzyme, wherein the pyridoxal acts as a cofactor). Then spontaneously, without the participation of enzymes, a Schiff base forms with spatially closed amino group of a lysine residue present in the other polypeptide chain. The resulting bond -N = CH- being a little resistant itself can be restored to a stable saturated bonds, becoming a very strong cross-link - lizinonorleucin.

In this technical solution, it may be assumed, when AFG compounds having primary amino groups introduced into the tissues of the sclera at a first process stage, they react with the allysine aldehyde groups in adjacent peptide and form a corresponding Schiff base with its subsequent reduction to a stable saturated structure.

In the second step of the process after AFG covalent immobilization depending on the nature of the functional groups remaining in the molecule, the following fundamental reactions leading to additional cross-linking of fibrillar collagen structures sclera are possible:
- aminoalcohols: free hydroxyl groups in their structure can react with aldehyde groups in the allysine in the adjacent collagen fibers to form acetal groups;
- aminoacids, oligo- and polypeptides, as well as polyamines: free amino groups in the composition of these compounds may react with the aldehyde groups of allysine in the adjacent collagen fibers;
- derivatives of 3-hydroxy-2-methylpyridine contain at least two functional groups: aldehyde, primary amine, alcohol groups that can react directly with the appropriate functional groups in adjacent collagen fibers.
- aminosaccharides: in a reactive aldose tautomeric form, in contrast to the inert pyranose ring form, they may react with amino groups in the side chains of lysine, arginine or hydroxylysine residues in the collagen fibers. Furthermore, the alcohol groups of these compounds may participate in cross-linking process with the aldehyde groups of al-lysine.

In addition, it can be assumed that the AFG used are stimulating the synthesis of collagen (procollagen) in fibroblasts - the cells producing it.

Thus, when using amines having functional groups, as might be expected, the effect of strengthening the sclera achieved due to a significant increase in the density of cross-links of collagen fibrillar structures of the sclera, despite the different nature of the functional groups of these cross-linking agents.

### Indications for the claimed drug administration

The indications for use of the claimed drug for strengthening the sclera is progressing myopia, especially in children and adolescents. Strengthening the sclera is carried out to stabilize the myopic process and prevent the development of complications in the eye fundus. During this age period, the intense myopia progression (at a high annual gradient, i.e. the increase in the degree of myopia of more than 1 diopter/yr.), accompanied by an abnormal dilation of scleral shell of the eye, leads to the appearance of complications, initially localized mainly in the periphery of the fundus .

With age, against the background of further progression of myopia, these changes apply to the central parts of the eye fundus, which leads to irreversible loss of vision, to a substantial restriction of the patient's work capacity and to disability. Strengthening the sclera in the early stages of the myopic process will prevent the development of serious complications. Using the non-invasive interventions for strengthening the sclera (for example, the monthly rate of instillation of the claimed preparation in the form of an aqueous solution) instead of the currently used scleroplastic operations is particularly important, given the children contingent, which is primarily indicated for sclera strengthening treatment.

The main advantages of using the claimed technical solutions are:
- high efficiency of the sclera strengthening, which significantly improves its mechanical stability and manifests in increasing its tensile strength and elasticity modulus;
- achievement of expressed sclera strengthening effect immediately after the end of treatment;
- easy and non-invasive treatment - the usual way of instillation, using eye gel or ophthalmic medicated film;
- no toxic or inflammatory effects throughout the course of drug treatment;
- no foreign body is entered in the area of the orbit and situated therein for a long time (foam), which excludes its possible damaging and irritating effect on the eyeball and the surrounding tissues of the orbit.

All these advantages of the invention are of key importance for clinical practice: when a simple non-invasive application achieves a high effect of sclera strengthening, allowing to stop the pathological process of straining the support shell of the eye, and thereby stabilizes the myopic process and prevents the development of pathological changes in the fundus associated with the progression of myopia.

### Industrial applicability

### Examples:

Example 1. In a glass flask, while stirring with a magnetic stirrer, there were dissolved 0.132 g (9.06 * 10⁻⁴ mol) D-lysine, Sigma in 10.0 ml balanced saline, then a solution of the amine as a neutralizing agent was added to a solution of 0.0652 g (9.06 * 10⁻⁴ mol) of polyacrylic acid (M_{w} 1800), Aldrich in 10.0 ml of distilled water and stirred for 15 min. Then an additional amount of polyacrylic acid was used to adjust the pH to 7.4.

Prepared 1.0 wt.% AFG salt solution is poured into glass bottles of 5 ml volume which sterilized by autoclaving at 121°C (1.1 atm) for 10 min.

This composition was used to strengthen the sclera. For this purpose, it is administered daily by instillation into the conjunctival sac of left eye "Chinchilla" breed rabbit, intact right eye was kept as a control. The treatment consisted of daily instillations, 2 times a day 1-2 drops within 1 month. During this period and for 1 month after the completion of instillation, the state of the rabbits eyes was controlled by biomicroscopy. No toxic or inflammatory effects were found. At the end of the observation period, the eyes were enucleated and used to prepare the samples of the sclera.

Example 2. In a glass flask with magnetic stirring there was dissolved 2.13 g (1.61 * 10⁻² mol) L-ornithine, Sigma in 70.0 ml of distilled water, and then dissolved amine was neutralized with 1.03 g (0.537 * 10⁻² mol) of citric acid dissolved in 26.0 ml of distilled water. Then an additional amount of sebacic acid was used to adjust the pH to 7.4. Prepared 3.3 wt.% AFG salt solution sterile filtered through a membrane Durapore^{®} (PVDF), Millipore with 0.22 micron pores and aseptically filled into glass bottles of volume 5 ml.

This composition was used to strengthen the sclera by instillation as described in Example 1.

Example 3. In a glass flask, while stirring with a magnetic stirrer, there was dissolved 0.803 g (0.554 * 10⁻² mol) spermidine, Sigma in 80.0 ml of distilled water, and then dissolved amine was neutralized with a 1.195 g (1.66 * 10⁻² mol) of polyacrylic acid (M_{w} 1800), Aldrich, dissolved in 18.0 ml of distilled water. Then an additional amount of polyacrylic acid was used to adjust the pH to 7.4. Prepared 2.0 wt.% AFG salt solution is sterile filtered through a Millipore membrane with 0.22 microns pores, and poured into glass bottles of volume 5 ml.

This composition was used to strengthen the sclera by instillation as described in Example 1.

**Example 4.** In a glass flask, while stirring with a magnetic stirrer, there was dissolved 0.437 g (2.17 * 10⁻³ mol) spermine, Sigma, in 70.0 ml of a balanced saline solution and then dissolved amine was neutralized with a 0,0633 g (4.34 * 10⁻³ mol) of adipic acid dissolved in 30.0 ml balanced saline solution. Then an additional amount of adipic acid was used to adjust the pH to 7.4. Prepared 0.5 wt.% AFG salt solution is sterile filtered through a Millipore membrane with 0.22 microns pores, and poured into glass bottles of volume 5 ml.

This composition was used to strengthen the sclera by instillation as described in Example 1.

### (Reference)

**Example 5.** In a glass flask, 0.15 g pyridoxamine dihydrochloride, Fluka during magnetic stirring was dissolved in 100.0 ml of distilled water and then added dropwise 5.0 wt.% aqueous solution of tris(hydroxymethyl)aminomethane to pH 7.2. Prepared 0.15 wt.% AFG salt solution is sterile filtered through a Millipore membrane with 0.22 microns pores, and poured into glass bottles of volume 5 ml.

This composition was used to strengthen the sclera by instillation as described in Example 1.

### (Reference)

**Example 6.** In a glass flask, while stirring with a magnetic stirrer, there was dissolved 4.20 g (2.54 * 10⁻³ mol) pyridoxine, Sigma in 50.0 ml of distilled water, and then dissolved amine was neutralized with a 1.78 g (2.54 * 10⁻³ mol) polyacrylic acid (M_{w} 1800), Aldrich, dissolved in 28.0 g of distilled water. Then an additional amount of polyacrylic acid solution was used to adjust the pH to 7.4. Prepared 7.0 wt.% AFG salt solution is sterile filtered through a Millipore membrane with 0.22 microns pores, and poured into glass bottles of volume 5 ml.

This composition was used to strengthen the sclera by instillation as described in Example 1.

### (Reference)

**Example 7.** In a glass flask 2.00 g of D-galactosamine hydrochloride, Sigma with magnetic stirring was dissolved in 98.0 ml of distilled water, followed by dropwise addition of 50 wt.% Aqueous solution of pyridoxine to pH 7.4. Prepared 2.0 wt.% AFG salt solution is sterile filtered through a Millipore membrane with 0.22 microns pores, and poured into glass bottles of volume 5 ml.

This composition was used to strengthen the sclera by instillation as described in Example 1.

**Example 8.** In a glass flask, 2.74 g of (1.88 * 10⁻² mol) D,L-lysine, Sigma with magnetic stirring was dissolved in 80.0 ml of distilled water, and then to the resulting solution 1.26 g (0.94 * 10⁻² mol) CuCl₂ dissolved in 16.0 ml of distilled water was added. Prepared 4.0 wt.% AFG complex compound solution having a pH of 6.8, was sterile filtered through a Millipore membrane with 0.22 microns pores, and poured into glass bottles of volume 5 ml.

This composition was used to strengthen the sclera by instillation as described in Example 1.

### (Reference)

**Example 9.** In a glass flask, 3.49 g (1.62 * 10⁻² mol) D-glucosamine hydrochloride, Sigma with magnetic stirring was dissolved in 60.0 ml of distilled water, and then to the resulting solution was added for 5 minutes 0.65 g (1.62 * 10⁻² mole) NaOH, dissolved in 16 ml of distilled water and then to the resulting free amine 1.09 g (0.812 * 10⁻² mol) CuCl₂ dissolved in 20 ml of distilled water was added. Prepared 4.0 wt.% AFG complex compound solution was sterile filtered through a Millipore membrane with 0.22 microns pores and dispensed in glass bottles of volume 5 ml.

This composition was used to strengthen the sclera by instillation as described in Example 1.

### (Reference)

**Example 10.** In a glass flask, 1.436 g (0.850 * 10⁻² mol) pyridoxine, Sigma with magnetic stirring was dissolved in 80.0 ml of distilled water and then to the solution 0.564 g (0.425 * 10⁻² mol) CuCl₂ dissolved in 19.0 ml of distilled water was added. Prepared 4.0 wt.% AFG complex compound solution was sterile filtered through a Millipore membrane with 0.22 microns pores, and poured into glass bottles of volume 5 ml.

This composition was used to strengthen the sclera by instillation as described in Example 1.

### (Reference)

**Example 11.** In a glass flask, 1.429 g (0.845 * 10⁻² mol) pyridoxine, Sigma with magnetic stirring was dissolved in 80.0 ml of distilled water and then to the solution 0.571 g (0.422 * 10⁻² mol) ZnCl₂ dissolved in 18.0 ml of distilled water was added. Prepared 4.0 wt.% AFG complex compound solution was sterile filtered through a Millipore membrane with 0.22 microns pores, and poured into glass bottles of volume 5 ml.

This composition was used to strengthen the sclera by instillation as described in Example 1.

**Example 12.** Gel based on cross-linked copolymer of acrylic acid and C10-30-alkylacrylates.

For preliminary mixing of 0.5 wt.% polymeric acid suspensions (gellant), 0.80 g of the cross-linked copolymer of acrylic acid and C10-30-alkylacrylates (Carbopol Ultrez 20, Noveon, Inc.) was added under vigorous mechanical stirring to 160 g of distilled water and stirred at room temperature for 15 min. To the resulting suspension, having a pH of 3.2, a solution of 0.96 g of tris(hydroxymethyl)aminomethane, Sigma was added to 14 g distilled water. In the resulting neutralization reaction, the used polymer acid (gellant) dissolves gradually passing into salt form, and after holding for 10 minutes a 1.0 wt.% the transparent viscous gel is formed having a pH 6.8. Next, the gel is sterilized by autoclaving at 121°C (1.1 atm) for 10 min. The resulting gel is directly used as an ophthalmic gel for treatment of myopia.

### (Reference)

**Example 13.** The gel obtained according to Example 12 is used as a gellant for the preparation of a composition further comprising AFG - 0.875 g (0.5 wt.%) D-galactosamine hydrochloride, Sigma (total concentration AFG - 1.5 wt%) which is used as an ophthalmic gel for treatment of myopia.

### (Reference)

**Example 14.** Preparation of a pharmaceutical formulation in the form of ophthalmic medicated film.

In the apparatus with a capacity of 1.0 liter, with stirring there are consistently loaded 95,0 g of ethanol, 180 g of a copolymer of acrylamide, vinylpyrrolidone and alkylacrylate (50/25/25) (reduced viscosity of 1.53 dl/g, water), obtained by the known method (see RF patent 2352589, 2007/2009 "The process for producing biosoluble polymers of acrylamide, vinylpyrrolidone and alkyl acrylate" / Belykh S.I. Davydov A.B. Mikhailov S.F., Khromov G.L. - [16]), and 4.8 g glycerol for 40 minutes introduced in 544.0 g of distilled water. The temperature in the apparatus is maintained in the range of 50-60°C. After 2 hr. the solution was cooled to 25°C, and 20.0 g D-glucosamine hydrochloride, Sigma (10,0 wt.) was added to the apparatus.%). Stirring for another 0.5 hours. Then the solution was applied evenly on the incorrodible substrate and dried at 60°C to form a polymeric strip or plate from which with a special die an oval films with smooth edges size 9.0 * 4.5 * 0.35 mm and an average weight of 15 mg were cutted. After this, film was laid out in individual packages and sterilized by radiation. The obtained OMF is used for treating myopia by placing in the conjunctival sac.

### References

1. Libman E.S., Ryazanov D.P., Kaleyeva E.V. "Disability as a result of visual impairment in Russia." V Russian national ophthalmology forum. Collection of scientific works of the scientific-practical conference with participants from abroad. Moscow, 2012, 2, 797-798;
2. Avetisov E.S. "Myopia". Moscow: 1999, 285 p.;
3. Iomdina E.N. "The biomechanical and biochemical sclera disorders with progressive myopia, and methods of their correction». Visual function in children and their correction/ Edited by Avetisova S.E., Kashchenko T.P., Shamshinova A.M. Moscow, 2006, 163-183;
4. Curtin B.J. «The Myopias: Basic science and clinical management». Harper and Row. Philadelphia. 1985;
5. Bailey A.J. intermediate labile inter molecular cross-links in collagen fibrils». Biochim. Biophys. Acta. 1968. v. 160, 447-453;
6. Tarutta E.P. "Sclera strengthening treatment and prevention of complications of progressive myopia" Visual function in children and their correction / Edited by S.E. Avetisov, T.P. Kashchenko, A.M. Shamshinova. Moscow, 2006, 191-202;
7. Ward B., Tarutta E. «The efficacy and safety of posterior pole buckles in the control of progressive high myopia. Eye (Lond). 2009 Dec. 23 (12), 2169-74;
8. Patent of the Russian Federation 2012336, 1987/1994. "The composition for the treatment of progressive myopia." Avetisov E.S., Vinetskaya M.I., Iomdina E.N. et al.;
9. Shilnikov L.V. Encyclopedia of clinical ophthalmology. M .: Scientific book, 2013, 380 p.;
10. Morozov V.I., Yakovlev A.A. Pharmacotherapy of eye diseases: a handbook. Publ. 3rd. M .: Medicine, 1998, 336 p.;
11. Iomdina E.N. "Biomechanics of the scleral shell of the eye with myopia: diagnostics of defects and their experimental correction". Thesis of doctor of biological sciences. M., 2000, 316 p.;
12. "Coordination chemistry" /Skopenko V.V., Tsivadze A.Y., Savranskii L.I., Garnovskii A.D. Moscow: ECC "Akademkniga", 2007, 487 p.;
13. Conato C., Contino A., Maccarrone G., Magr A., Remelli M., Tabb G. «Copper (II) complexes with L-lysine and L-ornithine: is the side-chain involved in the coordination. A thermodynamic and spectroscopic study. Thermochimica Acta, 362, 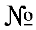 1, 2000, 13-23.;
14. Furmanova N.G., Berdalieva J.I., Chernaya T.S., Resnyansky V.F., Shyitieva N.K., Sulaimankulov K.S. "Synthesis and crystal structures of pyridoxine coordination compounds with zinc and cadmium sulphates." Crystallography. 2009, 54, 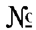 2, 255-261;
15. Bailey A. J., Paul R.G., Knott L. «Mechanism of maturation and ageing of collagen». Mech. Ag. Dev. 1998, 106, 1-56;
16. Patent of the Russian Federation 2352589, 2007/2009. "The method for producing biosoluble polymers of acrylamide, vinylpyrrolidone and alkyl acrylate". Belykh S.I., Davydov A.B., Mikhailov S.F., Khromov G.L.

## Claims

1. A pharmaceutical preparation for use in the prevention and treatment of progressive myopia, wherein the preparation is in the form of an aqueous solution for instillation or an ophthalmic gel or film dosage containing in its composition an effective amount of primary amine containing at least two primary amine groups in the form of salt or in composition of a complex compound of transition metal or mixtures thereof.

2. The preparation for use according to claim 1, wherein concentration of the salt or the complex compound of transition metal on the base of the primary amine is 0.15-7.0 wt.%.

3. The preparation for use according to claim 1, wherein the water media for solution is distilled water, or isotonic solution, or mixture thereof.

4. The preparation for use according to claim 1, wherein the water solution additionally contains gellant.

5. The preparation for use according to claim 1, wherein the primary amine is amino acids, or oligopeptides, or polypeptides, or polyamines, or a mixture thereof.

6. The preparation for use according to claim 5, wherein the amino acids are 2,4-diaminobutanoic acid or 2,5-diaminopentanoic acid (ornithine), or 2,6-diaminohexanoic acid (lysine), or 2,6-diamino-5-hydroxyhexanoic acid (hydroxylysine), or 2,7-diaminoheptanoic acid, or 2,8 diaminooctanoic acid, or 2,9-diaminononanoic acid, or 2, 10-diaminodecanoic acid, or 2,12-diaminododecanoic acid, or a mixture thereof.

7. The preparation for use according to claim 5, wherein the oligopeptides are oligopeptides having at least one link of L-ornithine, or L-lysine or L-hydroxylysine, or a mixture thereof.

8. The preparation for use according to claim 5, wherein the polypeptides are poly-L-ornithine, or poly-L-lysine, or poly-L-hydroxylysine, or poly-L-arginine, or a mixture thereof.

9. The preparation according to claim 1, wherein the salt is obtained by neutralization of the primary amine with acid.

10. The preparation for use according to claim11, wherein the acid is low molecular weight acid, polymeric acid, or their partially neutralized form, or mixtures thereof.

11. The preparation for use according to claim 1, wherein the transition metal is cation Cu²⁺ and/or Zn²⁺.

## Patentansprüche

1. Pharmazeutische Zubereitung zur Verwendung bei der Vorbeugung und Behandlung fortschreitender Myopie, wobei die Zubereitung in Form einer wässrigen Lösung zur Instillation oder einer ophthalmischen Gel- oder Filmdosis vorliegt, die in ihrer Zusammensetzung eine wirksame Menge von primärem Amin, das mindestens zwei primäre Amingruppen enthält, in Form eines Salzes oder in Zusammensetzung einer komplexen Verbindung von Übergangsmetall oder Mischungen davon enthält.

2. Zubereitung zur Verwendung nach Anspruch 1, wobei die Konzentration des Salzes oder der komplexen Verbindung von Übergangsmetall auf der Basis des primären Amins 0,15-7,0 Gew.-% beträgt.

3. Zubereitung zur Verwendung nach Anspruch 1, wobei das Wassermedium zur Lösung destilliertes Wasser oder isotonische Lösung oder eine Mischung davon ist.

4. Zubereitung zur Verwendung nach Anspruch 1, wobei die Wasserlösung zusätzlich Geliermittel enthält.

5. Zubereitung zur Verwendung nach Anspruch 1, wobei das primäre Amin Aminosäuren, Oligopeptide, Polypeptide, Polyamine oder eine Mischung davon ist.

6. Zubereitung zur Verwendung nach Anspruch 5, wobei die Aminosäuren 2,4-Diaminobutansäure oder 2,5-Diaminopentansäure (Ornithin) oder 2,6-Diaminohexansäure (Lysin) oder 2,6-Diamino-5-hydroxyhexansäure (Hydroxylysin) oder 2,7-Diaminoheptansäure oder 2,8-Diaminooctansäure oder 2,9-Diaminononansäure oder 2,10-Diaminodecansäure oder 2,12-Diaminododecansäure oder eine Mischung davon sind.

7. Zubereitung zur Verwendung nach Anspruch 5, wobei die Oligopeptide Oligopeptide mit mindestens einer Bindung aus L-Ornithin oder L-Lysin oder L-Hydroxylysin oder einer Mischung davon sind.

8. Zubereitung zur Verwendung nach Anspruch 5, wobei die Polypeptide Poly-L-Ornithin oder Poly-L-Lysin oder Poly-L-Hydroxylysin oder Poly-L-Arginin oder eine Mischung davon sind.

9. Zubereitung nach Anspruch 1, wobei das Salz durch Neutralisation des primären Amins mit Säure erhalten wird.

10. Zubereitung zur Verwendung nach Anspruch 11, wobei die Säure eine Säure mit niedrigem Molekulargewicht, Polymersäure oder deren teilweise neutralisierte Form oder Mischungen davon ist.

11. Zubereitung zur Verwendung nach Anspruch 1, wobei das Übergangsmetall Kation Cu²⁺ und/oder Zn²⁺ ist.

## Revendications

1. Préparation pharmaceutique pour utilisation dans la prévention et le traitement de myopie progressive, dans laquelle la préparation est sous la forme d'une solution aqueuse pour instillation ou d'un dosage de gel ou de film ophtalmique contenant, dans sa composition, une quantité efficace d'amine primaire contenant au moins deux groupes amine primaire sous la forme de sel ou dans une composition d'un composé complexe de métal de transition ou de mélanges de ceux-ci.

2. Préparation pour utilisation selon la revendication 1, dans laquelle la concentration du sel ou du composé complexe de métal de transition sur la base de l'amine primaire est de 0,15 à 7,0 % en poids.

3. Préparation pour utilisation selon la revendication 1, dans laquelle le milieu aqueux pour la solution est de l'eau distillée, ou une solution isotonique, ou un mélange de celles-ci.

4. Préparation pour utilisation selon la revendication 1, dans laquelle la solution aqueuse contient en outre un gélifiant.

5. Préparation pour utilisation selon la revendication 1, dans laquelle l'amine primaire est composée d'acides aminés, ou d'oligopeptides, ou de polypeptides, ou de polyamines, ou d'un mélange de ceux-ci.

6. Préparation pour utilisation selon la revendication 5, dans laquelle les acides aminés sont de l'acide 2,4-diaminobutanoïque ou de l'acide 2,5-diaminopentanoïque (ornithine), ou de l'acide 2,6-diaminohexanoïque (lysine), ou de l'acide 2,6-diamino-5-hydroxyhexanoïque (hydroxylysine), ou de l'acide 2,7-diaminoheptanoïque, ou de l'acide 2,8-diaminooctanoïque, ou de l'acide 2,9-diaminononanoïque, ou de l'acide 2,10-diaminodécanoïque, ou de l'acide 2,12-diaminododécanoïque, ou un mélange ceux-ci.

7. Préparation pour utilisation selon la revendication 5, dans laquelle les oligopeptides sont des oligopeptides présentant au moins une liaison de L-ornithine, ou de L-lysine ou de L-hydroxylysine, ou un mélange de celles-ci.

8. Préparation pour utilisation selon la revendication 5, dans laquelle les polypeptides sont la poly-L-ornithine, ou la poly-L-lysine, ou la poly-L-hydroxylysine, ou la poly-L-arginine, ou un mélange de celles-ci.

9. Préparation selon la revendication 1, dans laquelle le sel est obtenu par neutralisation de l'amine primaire avec un acide.

10. Préparation pour utilisation selon la revendication 11, dans laquelle l'acide est un acide à faible poids moléculaire, un acide polymère, ou leur forme partiellement neutralisée, ou des mélanges de ceux-ci.

11. Préparation pour utilisation selon la revendication 1, dans laquelle le métal de transition est le cation Cu²⁺ et/ou Zn²⁺.
